# EUROPEAN PATENT SPECIFICATION

(11) **EP 2 398 318 B1**
(45) Date of publication and mention of the grant of the patent: **05.12.2012**
(21) Application number: 10704724.3
(22) Date of filing: 19.02.2010
(51) Int. Cl.: A01N 37/52, A01N 43/50, A01N 47/34, A01P 7/04, A01N 25/02, A61L 9/01

(54) **IMPROVED-SCENT ECTOPARASITICIDAL FORMULATION**
EKTOPARASITIZIDE FORMULIERUNG MIT VERBESSERTEM GERUCH
COMPOSITION ECTOPARASITICIDE À PARFUM AMÉLIORÉ

(30) Priority: 23.02.2009 US 154520 P
(43) Date of publication of application: 28.12.2011
(73) Proprietor: Wyeth LLC, Madison, NJ 07940 (US)
(72) Inventor: FATTOHI, Nahla, Robbinsville New Jersey 08691 (US); GLIDDON, Michael, John, Lawrenceville New Jersey 08648 (US); SABNIS, Shobhan, Shashikant, Pennington New Jersey 08534 (US)
(74) Representative: Pfizer
(86) International application number: PCT/US2010/024677
(87) International publication number: WO 2010/096623

(56) References cited:
- WO-A1-2006/042099

## Description

### BACKGROUND OF THE INVENTION

Arthropod ectoparasites commonly infecting warm-blooded animals include ticks, mites, lice, fleas, blowflies, the ectoparasite *Lucilia* sp. of sheep, biting insects including keds (*Melophagus ovinus*) and migrating dipterous larvae such as *Hypoderma* sp. and *Dermatobia* in cattle, *Gastrophilus* in horses and *Cuterebra* sp. in rodents, dogs and cats. Metaflumizone, amitraz and demiditraz are useful for the prevention and control of ectoparasiticide infestations in warm-blooded animals. Topical administration is a preferred method for administration of these ingredients.

In order to provide topical formulations that are stable, easy to apply, spreadable and minimize run-off, existing formulations have relied on odoriferous solvents, which have given the formulations an inauspicious smell.

For example, WO 2006/042099 discloses a topical ectoparasiticidal composition comprising metaflumizone (R-28153). It further comprises a bridging agent (DMSO) and various carrier solvents (eucalyptol, 1-methoxy-2-propyl acetate, γ-hexalactone and N,N-diethyl-m-toluamide).

### SUMMARY OF THE INVENTION

The present invention provides an improved-scent ectoparasiticidal formulation, which is stable, easy-to-apply, spreadable and minimizes animal-runoff.

In one embodiment the present invention provides an improved-scent ectoparasiticidal composition, which comprises metaflumizone, fructone as the primary carrier solvent and optionally, amitraz or demiditraz. More particularly, the composition comprises metaflumizone, fructone, geranyl acetate as cosolvent and skin permeation enhancer and, optionally, additional carrier solvents and/or a bridging agent. More particularly, the composition comprises (on a w/v basis): about 20% to about 50% fructone; about 10% to about 30% metaflumizone; about 0% to about 20% amitraz or about 0% to about 20% demiditraz; about 5% to about 15% dimethylsulfoxide (DMSO); about 5% to about 15% N,N-diethyl-m-toluamide (DEET); about 5% to about 15% γ-hexalactone; about 5% to about 15% geranyl acetate; and about 1% to about 10% 1-methoxy-2-propyl acetate. More particularly, the composition comprises about 10% to about 20% w/v amitraz. Alternatively, the composition comprises about 10% to about 20% w/v demiditraz.

Another aspect of the invention provides a method for preventing or treating an ectoparasite infestation in a warm-blooded animal, comprising, topically administering to the warm-blooded animal the improved-scent ectoparasiticidal composition.

Another aspect of the invention provides a kit for preventing or treating an ectoparasite infestation in a warm-blooded animal comprising a topical unit dose formulation of the improved-scent ectoparasiticidal composition.

Other objects, features and advantages of the present invention will become apparent from the following detailed description.

### DETAILED DESCRIPTION OF THE INVENTION

Topical veterinary compositions containing metaflumizone, amitraz and/or demiditraz as active ingredients are highly desirable due to the effective and persistent activity of metaflumizone against a variety of ectoparasites, including, but not limited to, the dog flea, *Ctenocephalides canis,* and the efficacy of amitraz and demiditraz against ticks.

Existing "spot-on" applications of topical veterinary ectoparasiticidal compositions applied to the base of the neck of the animal aid in making the applied composition difficult for the animal to remove, but include solvents that provide less-favorable organoleptic properties. The limited solubility and stability of ectoparasiticide agents, particularly metaflumizone and amitraz, has heretofore required the inclusion of these solvents, such as cineole (eucalyptol), that give the formulation a less favorable odor.

Advantageously, the improved-scent composition of the present invention retains the desired physical characteristics over time, without loss of potency of the actives. Further, the composition of the invention exhibits sufficient viscosity to allow for the retention of the composition when applied topically to the animal's skin or hair, while facilitating the release of the ectoparasiticide(s), such as metaflumizone, amitraz and/or demiditraz, over the desired period of time.

In one embodiment, the improved-scent composition of the present invention comprises metaflumizone and fructone as the primary carrier component. In another embodiment, the composition further includes amitraz as an additional active. In another embodiment, the composition further includes demiditraz as an additional active. In another embodiment, the composition further comprises geranyl acetate. In another embodiment, the composition further comprises at least one of a bridging agent, skin permeation enhancer and/or a carrier solvent. Suitable carrier solvents include γ-hexalactone, N,N-diethyl-m-toluamide and 1-methoxy-2-propyl acetate. Suitable bridging agents are those such as, for example, dimethylsulfoxide.

In another embodiment, the improved-scent composition of the present invention comprises metaflumizone, fructone, geranyl acetate, and at least one, at least two, or at least three of γ-hexalactone, N,N-diethyl-m-toluamide, 1-methoxy-2-propyl acetate and dimethylsulfoxide, and further optionally including amitraz and/or demiditraz.

In another embodiment, the metaflumizone is present at about 10% to about 40% w/v, or about 10% to about 30% w/v, or about 10% to about 20% w/v, or about 15% w/v. In another embodiment, the amitraz is present at about 0% to about 20% w/v, or about 10% to about 20% w/v, or about 15% w/v. In another embodiment, the demiditraz is present at about 0% to about 20% w/v, or about 10% to about 20% w/v, or about 15% w/v. In another embodiment, the fructone is present at about 20% to about 60% w/v, or about 20% to about 50% w/v, or about 20% to about 40% w/v, or about 30% w/v. In another embodiment, the geranyl acetate is present at about 5% to about 15% w/v, or about 7.5% to about 13% w/v, or about 10% w/v. In another embodiment, γ-hexalactone is present at about 5% to about 15% w/v, N,N-diethyl-m-toluamide is present at about 5% to about 15% w/v and 1-methoxy-2-propyl acetate is present at about 0% to about 15% w/v, or about 5% to about 15% w/v, or about 1% to about 10% w/v, or about 5% w/v. In another embodiment, dimethylsulfoxide is present at about 5% to about 15% w/v, or about 10% w/v. In another embodiment, the composition comprises, on a w/v basis, about 15% amitraz, about 15% metaflumizone, about 20% to about 40% fructone and about 10% geranyl acetate. In another embodiment, the composition comprises, on a w/v basis, about 15% demiditraz, about 15% metaflumizone, about 20% to about 40% fructone and about 10% geranyl acetate. In another embodiment, the composition comprises, on a w/v basis, about 15% to about 25% metaflumizone, about 20% to about 50% fructone and about 10% geranyl acetate. In another embodiment, the composition comprises, on a w/v basis, about 10% dimethyl sulfoxide, about 10% γ-hexalactone, about 10% N,N-diethyl-m-toluamide and about 5% 1-methoxy-2-propyl acetate.

In another embodiment, the improved-scent composition of the present invention comprises (on a w/v basis): about 20% to about 40% fructone; about 10% to about 20% metaflumizone; about 5% to about 15% DMSO; about 5% to about 15% N,N-diethyl-m-toluamide; about 5% to about 15% γ-hexalactone; about 5% to about 15% geranyl acetate; and about 0% to about 10% 1-methoxy-2-propyl acetate. More particularly, the composition further comprises about 10% to about 20% amitraz. Alternatively, the composition further comprises about 10% to about 20% demiditraz. In another embodiment, the composition consists essentially of (on a w/v basis), about 25% to about 35% fructone, about 15% amitraz, about 15% metaflumizone, about 10% dimethylsulfoxide, about 10% N,N-diethyl-m-toluamide, about 10% γ-hexalactone, about 10% geranyl acetate and about 5% 1-methoxy-2-propyl acetate. In another embodiment, the composition consists essentially of (on a w/v basis), about 25% to about 35% fructone, about 15% demiditraz, about 15% metaflumizone, about 10% dimethylsulfoxide, about 10% N,N-diethyl-m-toluamide, about 10% γ-hexalactone, about 10% geranyl acetate and about 5% 1-methoxy-2-propyl acetate.

In another embodiment the composition comprising, on a w/v basis, about 25% to about 50% fructone; about 10% to about 30% metaflumizone; about 5% to about 15% DMSO; about 5% to about 15% N,N-diethyl-m-toluamide; about 5% to about 15% γ-hexalactone; about 5% to about 15% geranyl acetate; and 0% to about 10% 1-methoxy-2-propyl acetate.

Another aspect of the invention provides a method for preventing or treating an ectoparasite infestation in a warm-blooded animal, comprising, topically administering to the warm-blooded animal the improved-scent composition described herein. More particularly, said composition is administered in one or more spots or discrete areas on the back of the warm-blood animal. In another embodiment, the ectoparasiticide is applied to the animal in 1, 2, 3, 4, or 5 locations (spots), preferably down the back of the animal or at the base of the neck. More particularly, the warm-blooded animal is a dog. More particular still, the dog receives about 15 mg/kg to about 25 mg/kg, preferably about 20 mg/kg of each of amitraz and metaflumizone. Alternatively, the dog receives about 15 mg/kg to about 25 mg/kg, preferably about 20 mg/kg of each of demiditraz and metaflumizone.

Another aspect of the invention provides a method for preventing or treating an ectoparasite infestation in a warm-blooded animal, comprising administering a diluted form of the improved-scent composition as a pour-on. More particularly, the diluted form of the improved-scent composition comprises about 1% to about 95% of the improved-scent composition described herein and at least one other solvent.

Another aspect of the invention provides a kit for preventing or treating an ectoparasite infestation in a warm-blooded animal comprising a topical unit dose formulation of the improved-scent composition described herein.

In another embodiment, the warm-blooded animal is a cat. In a still further embodiment, the warm-blooded animal is a farm animal. In another embodiment, the warm-blooded animal is a horse.

In another embodiment, the bridging agent is selected from the group consisting of an alkyl methylsulfoxide, e.g. dimethylsulfoxide, decylmethyl sulfoxide or tetradecylmethylsulfoxide, a pyrrolidone, e.g., 2-pyrrolidone, N-methyl-2-pyrrolidone or N-(2-hydroxyethyl)pyrrolidone, a laurocapram, acetone, dimethyl acetamide, dimethylformamide, and tetrahydrofurfuryl alcohol. In another embodiment, the bridging agent is selected from the group consisting of an L-amino acid, dimethylsulfoxide (DMSO) and a fatty acid.

In another embodiment, the surfactant is an anionic or cationic surfactant, a non-ionic surfactant, alcohol alkoxylate, or a nonylphenol ethoxylate.

In another embodiment, the composition comprises a diluent, an adjuvant, an excipient, a preservative, or a vehicle with which the active (e.g. metaflumizone and/or amitraz or demiditraz) is administered. In another embodiment, the composition includes petroleum oil, animal oil, vegetable oil, peanut oil, soybean oil, mineral oil, and sesame oil. In another embodiment, the composition does not comprise eucalyptol. In another embodiment, the composition further comprises a preservative selected from the group consisting of methylparaben, propylparaben, thiomersal, and EDTA.

Bridging agents that are suitably employed in the compositions disclosed herein include, without limitation, alkyl methyl sulfoxides (such as dimethylsulfoxide (DMSO), decylmethyl sulfoxide and tetradecylmethyl sulfoxide); pyrrolidones (such as 2-pyrrolidone, N-methyl-2-pyrrolidone and N-(2-hydroxyethyl) pyrrolidone); laurocapram; and miscellaneous solvents such as acetone, dimethyl acetamide, dimethyl formamide, and tetrahydrofurfuryl alcohol. Other bridging agents include amphiphiles such as L-amino acids, and fatty acids. Additional bridging agents are disclosed in Remington: The Science and Practice of Pharmacy, 19th Edition (1995), on page 1583.

The term "carrier" refers to a diluent, adjuvant, excipient, preservative, and/or vehicle with which a compound or composition is administered. Carrier solvents that may be suitably employed in the composition of the invention include single solvents, or a mixture of two or more solvents. Preferably, the carrier solvents have minimal odor or mask odors of other agents. Due to the instability of metaflumizone and amitraz in the presence of primary alcohols, preferred solvents are non-hydroxyl-group-containing solvents. Surprisingly, the use of fructone as a carrier solvent provides optimum formulation properties while minimizing odors. In addition to fructone, γ-hexalactone (also known as γ-caprolactone; ethyl butyrolactone; γ-ethyl-n-butyrolactone; hexanolide-1, 4; 4-hydroxy hexanoic acid γ-lactone or tonkalide), δ-hexalactone, γ-butyrolactone etc., Synperonic NCA 830, can be employed. Within other embodiments, solvents such as N,N-diethyl-m-toluamide, dimethyl isosorbide, diisopropyl adipate and/or 1-methoxy-2-propyl acetate may be advantageously utilized in combination with the fructone, γ-hexalactone to comprise the carrier solvent mixture. Suitable pharmaceutical carriers are described in "Remington's Pharmaceutical Sciences" by E.W. Martin, 18th Edition.

As used in the specification and claims, the terms "about" and "approximately" designate that a value is within a statistically meaningful range. Such a range can be typically within 10%, more typically still within 5%, and even more typically within 2% of a given value or range. The allowable variation encompassed by the terms "about" and "approximately" depends on the particular system under study, and can be readily appreciated by one of ordinary skill in the art.

As used herein, the term "w/v" designates weight of component/volume of composition, and the term "mg/kg" designates milligrams per kilogram of body weight. The term "a.i." or "ai" designates active ingredient, and may be combined with other terms. For example "mg a.i./kg" designates milligrams of active ingredient per kilogram of body weight.

As used herein, the term "treating" or "treatment" of a condition, such as an ectoparasite infestation, includes inhibiting an existing condition or arresting its development; or ameliorating or causing regression of the condition. The term "preventing" or "prevention" of a condition, such as an ectoparasite infestation, includes substantially blocking or inhibiting the development or growth of a condition before it starts. Compositions that treat or prevent infestations herein will preferably exhibit at least 90% efficacy.

As used herein, the term "ectoparasiticide" refers to an agent that is capable of preventing, reducing or eliminating ectoparasite infestations. Preferred ectoparasiticides of the present invention include metaflumizone, amitraz, demiditraz, fipronil, pyriprole, dinotefuran and imidacloprid.

As used herein, the term "organoleptic" refers to the sensory properties of a particular composition, particularly the smell, taste, colour, and feel of the composition. Improved or enhanced organoleptic properties of ectoparasiticidal compositions, as used herein, generally refers to reduced scent or a favorable scent.

Metaflumizone is known in the art by its chemical name: (E,Z)-2-[2-(4-cyanophenyl)-1-[3-(trifluoromethyl)phenyl]ethylidene]-N-[4-(tri-fluoromethoxy)-phenyl]hydrazinecarboxamide and is described in U.S. 5,543,573. The chemical structure of metaflumizone is shown below:

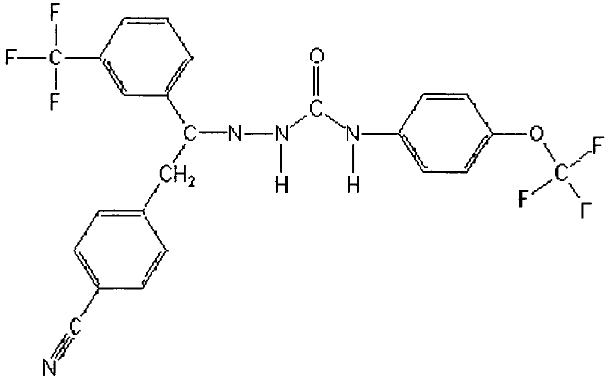

ProMeris^{®} is the trade name for the veterinary formulation of metaflumizone (plus amitraz for dogs) marketed by Fort Dodge Animal Health (Wyeth, now Pfizer).

Demiditraz is known in the art by its chemical name: 2-[(1S)-1-(2,3-Dimethylphenyl)ethyl]-1H-imidazole and is described in Example 58 of U.S. Publication No. 2007/0167506.

To manufacture the improved-scent compositions of the present invention, metaflumizone and optionally amitraz and/or demiditraz may be dissolved in the carrier solvent or solvents and bridging agent, and the surfactant, if desired, added to the carrier solvent solution. These compositions may then be utilized for spot-on topical application or may be further diluted for alternative uses. Compositions are most suitably formulated as creams, gels, solutions, or in microspheres.

Improved-scent ectoparasiticidal compositions of the invention may further comprise other agents known in the art, such as preservatives (e.g., methylparaben and propylparaben), colorants, antioxidants, or the like. Generally, these agents are present in the composition in an amount up to about 2% on a weight to volume basis. Preservatives may include, for example, thiomersal, EDTA, or the like.

Suitable exemplary polymers ("polymeric agents") for gelling and/or adhering that may be used in the compositions of the invention include, but are not limited to, colloidal silicone dioxide, ethyl cellulose, methyl cellulose, methacrylic esters copolymers, carboxylated vinyl acetate terpolymer, Resyn^{®} 29-2930, and polyvinylpropylene (PVP)/Vinyl acetate copolymers. "Gantrez"^{®} is the trade name for a family of polyvinylmethylether formulations (as solutions, creams, powders, etc.) manufactured by International Specialty Products, of Wayne, New Jersey. Gantrez powder formulations comprise poly(vinylmethylether/maleic anhydride). Gantrez cream formulations comprise ethyl or butyl esters of polyvinylmethylether/maleic anhydride copolymer and ethyl or butyl esters of PVM/MA copolymer. Resyn^{®} 29-2930 is the trade name for vinyl acetate / crotanoates / vinylneodecanoate copolymer.

Carbopol^{®} is the trade name of exemplary carbomers used in the formulation of certain embodiments of the presently disclosed compositions. Acrylic acid polymers other than-or in addition to-carbomers are also contemplated for use in the present invention.

In those embodiments in which compositions are formulated as microspheres, polymers including, but not limited to, sulfopolyester (AQ55S polymer from Eastman Chemical Co., Kingsport, Tenn., USA; typical repeating units of these polymers are disclosed in column 7 of U.S. 5,260,052.), and cellulose acetate butyrate (CAB), poly(lactide-co-glycolide) (PLGA) may be employed. Alternatively, microsphere formulation may employ one or more of polylactic galactide, hollow microspheres such as Expancel (Nobel Industrie), Polytrap (Dow Coming), and hollow silica microspheres (Silica Beads from Maprecos).

Additionally, UV-absorbing compounds, photostabilizers, viscosity modifying agents, antimicrobial agents, dyes, thickeners, antioxidants, taste enhancers or deterrents, vitamins, adherents, perfumes, deodorants, physiologically or dermatologically acceptable carriers, diluents, excipients or adjuvants may be included in the compositions and formulations of the present invention.

Advantageously, the ectoparasiticidal topical veterinary composition of the invention allows for administration of active ingredients and demonstrates no irritation and minimal adverse scent to the skin/hide/hair of the host animal with reduced and improved organoleptic properties.

When topically administered, the improved-scent ectoparasiticidal composition of the invention is effective for preventing or mitigating ectoparasitic infection and/or infestation for prolonged periods of time in warm-blooded animals such as swine, cattle, sheep, horses, goats, camels, water buffalo, bison, donkeys, rabbits, kangaroos, fallow deer, reindeer, minks, chinchillas, raccoons, chicken, geese, turkeys, ducks, dogs, cats, or the like, preferably dogs, cats, swine, cattle, horses or sheep, and most preferably dogs.

Examples of topical administrations suitable for use in the method of the invention include spot-on, pour-on, dip, wash, shampoo, foam, gel, lotion, cream, microsphere-encapsulated formulation, powder, or any of the conventional means of topically applying a liquid or semi-liquid veterinary composition. The topical mode of administration will vary with the species and size of the host animal. In a preferred embodiment, the composition of the present invention is administered as a spot-on formulation to a dog.

Ectoparasitic infection or infestations suitable for treatment by the methods of the invention include fleas, ticks, lice, mites and flies. In particular, the methods and kits of the present invention are suitable for treating or preventing flea infestations, and even more particularly, infestations of the dog flea, *Ctenocephalides canis* and tick infestations.

The composition of the invention may be administered in dose rates of mg of active ingredient per kg of body weight of the host animal. As will be understood by those skilled in the art, dose rates suitable for use in the method of invention will vary depending upon the identity of the ectoparasiticide, the mode of administration, the species and health of the host animal, the target parasite, the degree of infection or infestation, the breeding habitat, the potency of the additional parasiticidal compound, or the like.

Typical "spot-on" applications of exemplary embodiments of the invention are applied to the base of the neck of the animal or generally along the dorsal midline in the area between the shoulder blades, so as to aid in making the applied composition difficult for the animal to remove.

For a more clear understanding of the invention, the following examples are set forth hereinbelow. These examples are illustrative.

Unless otherwise designated, all parts are parts by weight/volume. The term qs designates quantity sufficient to obtain a total of 100%.

### EXAMPLES

### LOW-SCENT METAFLUMIZONE-AMITRAZ SOLUTION, SUITABLE FOR USE AS A SPOT-ON TREATMENT

### COMPOSITION A:

| | |
|---|---|
| | 15% w/v Metaflumizone/15% w/v Amitraz Spot-On - Alternate Formula |
| Volume to be applied per 10 kg body weight | 1.34 mL |

| INGREDIENTS | Quantities (% w/v) |
|---|---|
| Metaflumizone^{†} (100% pure) | 15.3 |
| Amitraz^{†} (100% pure) | 15.3 |
| N,N-Diethyl-*m*-toluamide | 10.0 |
| Dimethyl sulfoxide | 10.0 |
| 1-Methoxy-2-propyl acetate | 5.00 |
| Geranyl acetate | 10.0 |
| γ-Hexalactone | 10.0 |
| Fructone | q.s. ad (31.4) |
| Density (g/mL) | 1.070 |

| | |
|---|---|
| ^{†}Contains 2% overage. | |

In a vessel containing fructone, γ-hexalactone, followed by N,N-diethyl-m-toluamide, dimethyl sulfoxide (DMSO), 1-methoxy-2-propyl acetate, and finally geranyl acetate are added and agitated until mixed. Metaflumizone and amitraz are then added and the solution is thoroughly mixed until all solids are completely dissolved. The solution is then mixed over 4Å activated molecular sieves, filtered and packaged.

The following compositions are prepared according to the general procedure described fro composition A.

### COMPOSITION B:

| | |
|---|---|
| | 15% w/v Metaflumizone/15% w/v Amitraz Spot-On - Alternate Formula |
| Volume to be applied per 10 kg body weight | 1.34 mL |

| INGREDIENTS | Quantities (% w/v) |
|---|---|
| Metaflumizone^{†} (100% pure) | 15.3 |
| Amitraz^{†} (100% pure) | 15.3 |
| N,N-Diethyl-*m*-toluamide | 10.0 |
| Dimethyl sulfoxide | 10.0 |
| 1-Methoxy-2-propyl acetate | 5.00 |
| Geranyl acetate | 10.0 |
| Fructone | q.s. ad (42.2) |
| Density (g/mL) | 1.070 |

| | |
|---|---|
| ^{†} Contains 2% overage. | |

### COMPOSITION C:

| | |
|---|---|
| | 15% w/v Metaflumizone Canine Flea only Spot-On - Alternate Formula |
| Volume to be applied per | |
| 10 kg body weight | |

| INGREDIENTS | Quantities (% w/v) |
|---|---|
| Metaflumizone^{†} (100% pure) | 15.3 |
| N,N-Diethyl-*m*-toluamide | 13.8 |
| Dimethyl sulfoxide | 10.0 |
| 1-Methoxy-2-propyl acetate | 5.00 |
| Geranyl acetate | 10.0 |
| γ-Hexalactone | 10.0 |
| Fructone | q.s. ad (43.2) |
| Density (g/mL) | 1.073 |

| | |
|---|---|
| ^{†}Contains 2% overage. | |

### COMPOSITION D:

| | |
|---|---|
| | 25% w/v Metaflumizone Canine Flea only Spot-On |
| Volume to be applied per 10 kg body weight | |

| INGREDIENTS | Quantities (% w/v) |
|---|---|
| Metaflumizone^{†} (100% pure) | 25.50 |
| N,N-Diethyl-*m*-toluamide | 13.8 |
| Dimethyl sulfoxide | 10.0 |
| 1-Methoxy-2-propyl acetate | 5.00 |
| Geranyl acetate | 10.0 |
| γ-Hexalactone | 10.0 |
| Fructone | q.s. ad (35.3) |
| Density (g/mL) | 1.096 |

| | |
|---|---|
| ^{†}Contains 2% overage. | |

### COMPOSITION E:

| | |
|---|---|
| | 15% w/v Metaflumizone/15% w/v Amitraz Spot-On - Alternate Formula |
| Volume to be applied per 10 kq body weigh | 1.34 mL |

| INGREDIENTS | Quantities (% w/v) |
|---|---|
| Metaflumizone^{†} (100% pure) | 15.3 |
| Amitraz^{†} (100% pure) | 15.3 |
| N,N-Diethyl-*m*-toluamide | 10.0 |
| Dimethyl sulfoxide | 10.0 |
| 1-Methoxy-2-propyl acetate | 5.0 |
| Orange Flower Ether | 10.0 |
| γ-Hexalactone | 10.0 |
| Fructone | q.s. ad (31.4) |
| Density (g/mL) | 1.070 |

| | |
|---|---|
| ^{†}Contains 2% overage. | |

### COMPOSITION M:

| | |
|---|---|
| | 15% w/v Metaflumizone/15% w/v Demiditraz Spot-On Formula |
| Volume to be applied per 10 kg body weight | 1.34 mL |

| INGREDIENTS | Quantities (% w/v) |
|---|---|
| Metaflumizone (100% pure) | 15 |
| Demiditraz | 15 |
| N,N-Diethyl-*m*-toluamide | 10.0 |
| Dimethyl sulfoxide | 10.0 |
| 1-Methoxy-2-propyl acetate | 5.0 |
| Orange Flower Ether | 10.0 |
| γ-Hexalactone | 10.0 |
| Fructone | q.s. ad |

### COMPOSITION N:

| | |
|---|---|
| | 20% w/v Metaflumizone Spot-On Formula |
| Volume to be applied per 10 kg body weight | 1.34 mL |

| INGREDIENTS | Quantities (% w/v) |
|---|---|
| Metaflumizone (100% pure) | 20 |
| N,N-Diethyl-*m*-toluamide | 10.0 |
| Dimethyl sulfoxide | 10.0 |
| 1-Methoxy-2-propyl acetate | 5.0 |
| Orange Flower Ether | 10.0 |
| γ-Hexalactone | 10.0 |
| Fructone | q.s. ad |

### Organoleptic studies:

The organoleptic studies were performed on different compositions to determine the acceptability of the formulation's perceived odor in comparison to that of the original ProMeris^{®} for dog spot-on.

### Organoleptic Test 1: Total number of people = 40

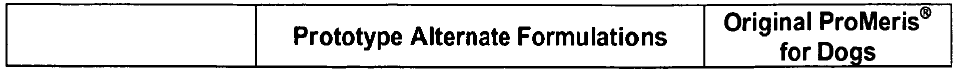

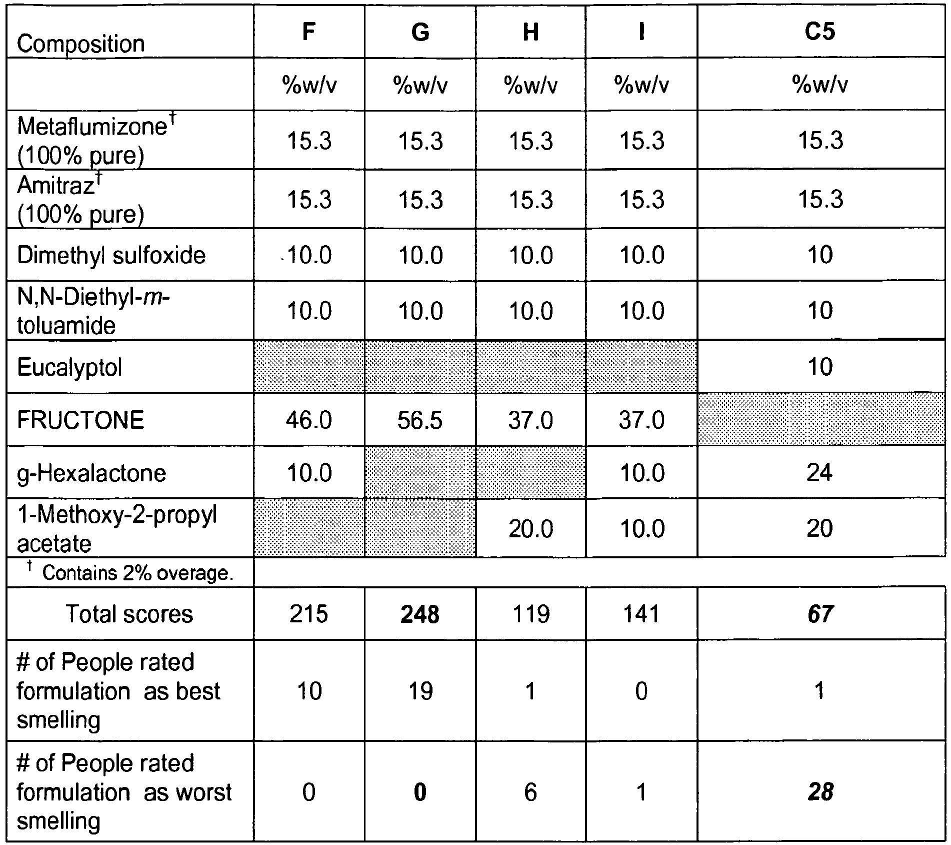

A small volume (1.0 ml) of each composition was placed on a filter paper in a petri dish. 40 persons employed by Wyeth volunteered to take part in the organoleptic test. Each volunteer smelled the sample and then rated the odor from 1 to 7, with a score of 7 being the best. The rating was such that the higher the score, the more acceptable the perceived odor was. Results indicated that composition G scored the highest aggregate of 248, which means it had the most acceptable odor. Also, zero people out of 40 scored it as the worst smelling formulation. Composition G contained high levels of fructone, which was observed to have a fruity odor.

### Organoleptic Test 2: Total number of people = 36

Organoleptic test was performed on different variations of the preferred composition (G) from test 1, with geranyl acetate and orange flower ether evaluated as possible partial replacements for 1-Methoxy-2-propyl acetate. 36 persons employed by Wyeth volunteered to take part in organoleptic test 2.

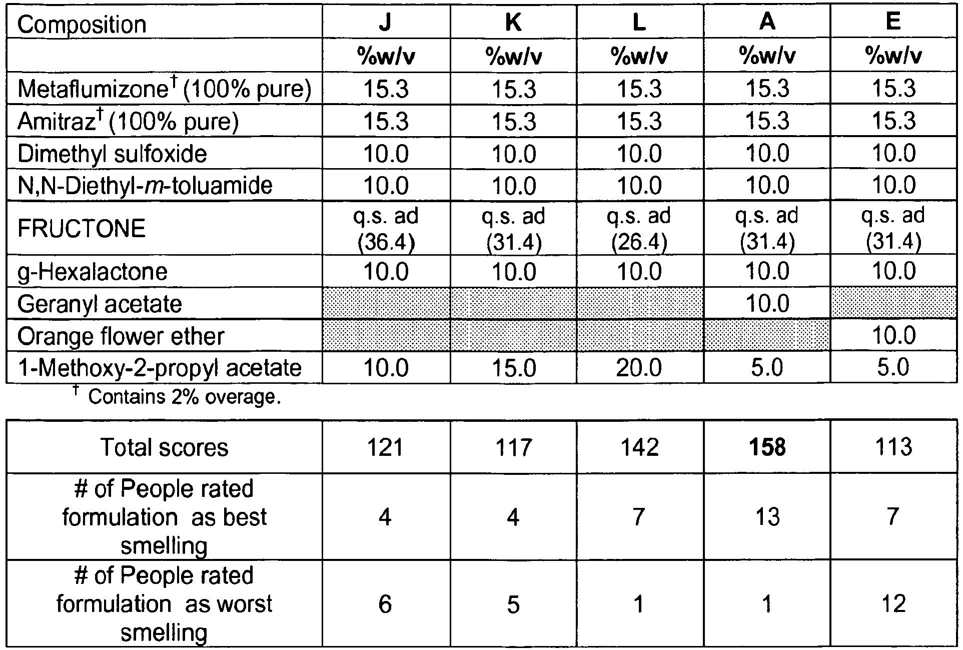

Results show that composition A scored a highest aggregate of 158.

### Efficacy study:

Compositions A and B were tested for efficacy to determine if the new spot-on formulations are as efficacious as ProMeris^{®} for controlling existing infestation and weekly challenges of flea and tick infestations on mixed breed dogs. Results showed that all dogs presented normal health and behavior throughout the course of study. Flea counts indicated that efficacy rates against infestation for Compositions A, B, and ProMeds^{®} for Dogs, were not statistically different and ≥ 98.8 % on all time points evaluated after treatment up to Day 42. In regards to tick counts, geometric means were not statistically different between Compositions A, B, and ProMeris® for Dogs and ≥ 97.3% on all times evaluated after treatment up to Day 28. On study day 35, efficacy rate for Composition B dropped below 90% and was significantly lower than Composition A and ProMeris® for Dogs.

## Claims

1. An ectoparasiticidal composition comprising metaflumizone and fructone.

2. The composition of claim 1, wherein metaflumizone is present at about 10% to about 40% w/v.

3. The composition of claim 1 or 2, wherein fructone is present at about 20% to about 60% w/v.

4. The composition of any one of claims 1-3, further comprising geranyl acetate.

5. The composition of claim 4, wherein geranyl acetate is present at about 5% to about 15% w/v.

6. The composition of any one of claims 1-5, further comprising at least one of a bridging agent, skin permeation enhancer and/or a carrier solvent.

7. The composition of claim 6, wherein the bridging agent is dimethysulfoxide (DMSO).

8. The composition of claim 7, wherein DMSO is present at about 5% to about 15% w/v.

9. The composition of any one of claims 6-8, wherein the carrier solvent is selected from the group consisting of γ-hexalactone, N,N-diethyl-m-toluamide and 1-methoxy-2-propyl acetate.

10. The composition according to any one of claims 1-9 comprising, on a w/v basis, about 25% to about 50% fructone;
about 10% to about 30% metaflumizone;
about 5% to about 15% DMSO;
about 5% to about 15% N,N-diethyl-m-toluamide;
about 5% to about 15% γ-hexalactone;
about 5% to about 15% geranyl acetate; and
0% to about 10% 1-methoxy-2-propyl acetate.

11. The composition of any one of claims 1-9, further comprising amitraz.

12. The composition of any one of claims 1-9, further comprising demiditraz.

13. A composition according to any one of claims 1-12 for use as a medicament for preventing or treating an ectoparasite infestation in a warm-blooded animal.

14. The composition of claim 13, wherein the medicament is adapted for topical administration in at least one spot on the back of the warm-blood animal.

15. A kit for preventing or treating an ectoparasite infestation in a warm-blooded animal comprising a topical unit dose of a composition of any one of claims 1-12.

## Patentansprüche

1. Ectoparasitizide Zusammensetzung, die Metaflumizon und Fructone umfasst.

2. Zusammensetzung gemäß Anspruch 1, wobei Metaflumizon mit etwa 10% bis etwa 40% G/V vorliegt.

3. Zusammensetzung gemäß Anspruch 1 oder 2, wobei Fructone mit etwa 20% bis etwa 60% G/V vorliegt.

4. Zusammensetzung gemäß einem der Ansprüche 1-3, die weiterhin Geranylacetat umfasst.

5. Zusammensetzung gemäß Anspruch 4, wobei Geranylacetat mit etwa 5% bis etwa 15% G/V vorliegt.

6. Zusammensetzung gemäß einem der Ansprüche 1-5, die weiterhin mindestens eines von einem Verbrückungsmittel, einem Mittel zur Verbesserung der Hautpermeation und/oder einem Trägerlösemittel umfasst.

7. Zusammensetzung gemäß Anspruch 6, wobei das Verbrückungsmittel Dimethylsulfoxid (DMSO) ist.

8. Zusammensetzung gemäß Anspruch 7, wobei DMSO mit etwa 5% bis etwa 15% G/V vorliegt.

9. Zusammensetzung gemäß einem der Ansprüche 6-8, wobei das Trägerlösemittel ausgewählt ist aus der Gruppe, bestehend aus γ-Hexalacton, N,N-Diethyl-m-toluamid und 1-Methoxy-2-propylacetat.

10. Zusammensetzung gemäß einem der Ansprüche 1-9, die auf einer G/V-Basis umfasst
etwa 25% bis etwa 50% Fructone;
etwa 10% bis etwa 30% Metaflumizon;
etwa 5% bis etwa 15% DMSO;
etwa 5% bis etwa 15% N,N-Diethyl-m-toluamid;
etwa 5% bis etwa 15% γ-Hexalacton;
etwa 5% bis etwa 15% Geranylacetat; und
0% bis etwa 10% 1-Methoxy-2-propylacetat.

11. Zusammensetzung gemäß einem der Ansprüche 1-9, die weiterhin Amitraz umfasst.

12. Zusammensetzung gemäß einem der Ansprüche 1-9, die weiterhin Demiditraz umfasst.

13. Zusammensetzung gemäß einem der Ansprüche 1-12 zur Verwendung als ein Medikament zur Vorbeugung oder Behandlung eines Befalls mit Ectoparasiten in einem warmblütigen Tier.

14. Zusammensetzung gemäß Anspruch 13, wobei das Medikament für eine topische Verabreichung an mindestens einer Stelle auf dem Rücken des warmblütigen Tiers angepasst ist.

15. Kit zur Vorbeugung oder Behandlung eines Befalls mit Ectoparasiten in einem warmblütigen Tier, das eine topische Einzeldosis einer Zusammensetzung gemäß einem der Ansprüche 1-12 umfasst.

## Revendications

1. Composition ectoparasiticide comprenant de la métaflumizone et de la fructone.

2. Composition selon la revendication 1, dans laquelle la métaflumizone est présente à raison d'environ 10 % à environ 40 % en pds/v.

3. Composition selon la revendication 1 ou 2, dans laquelle la fructone est présente à raison d'environ 20 % à environ 60 % en pds/v.

4. Composition selon l'une quelconque des revendications 1 à 3, comprenant en outre de l'acétate de géranyle.

5. Composition selon la revendication 4, dans laquelle l'acétate de géranyle est présent à raison d'environ 5 % à environ 15 % en pds/v.

6. Composition selon l'une quelconque des revendications 1 à 5, comprenant en outre au moins l'un parmi un agent de pontage, un agent augmentant la perméabilité de la peau et/ou un solvant formant support.

7. Composition selon la revendication 6, dans laquelle l'agent de pontage est le diméthylsulfoxyde (DMSO).

8. Composition selon la revendication 7, dans laquelle le DMSO est présent à raison d'environ 5 % à environ 15 % en pds/v.

9. Composition selon l'une quelconque des revendications 6 à 8, dans laquelle le solvant formant support est sélectionné dans le groupe consistant en la γ-hexalactone, le N,N-diéthyl-m-toluamide et l'acétate de 1-méthoxy-2-propyle.

10. Composition selon l'une quelconque des revendications 1 à 9, comprenant, sur la base du pds/v,
d'environ 25 % à environ 50 % de fructone ;
d'environ 10 % à environ 30 % de métaflumizone ;
d'environ 5 % à environ 15 % de DMSO ;
d'environ 5 % à environ 15 % de N,N-diéthyl-m-toluamide ;
d'environ 5 % à environ 15 % de γ-hexalactone ;
d'environ 5 % à environ 15 % d'acétate de géranyle ; et
de 0 % à environ 10 % d'acétate de 1-méthoxy-2-propyle.

11. Composition selon l'une quelconque des revendications 1 à 9, comprenant en outre de l'amitraz.

12. Composition selon l'une quelconque des revendications 1 à 9, comprenant en outre du démiditraz.

13. Composition selon l'une quelconque des revendications 1 à 12 en vue d'une utilisation en tant que médicament pour la prévention ou le traitement d'une infestation par des ectoparasites chez un animal à sang chaud.

14. Composition selon la revendication 13, dans laquelle le médicament est adapté à l'administration topique par au moins un dépôt ponctuel sur le dos de l'animal à sang chaud.

15. Kit de prévention ou de traitement d'une infestation par des ectoparasites chez un animal à sang chaud, comprenant une dose unitaire topique d'une composition selon l'une quelconque des revendications 1 à 12.
